# DEMANDE DE BREVET EUROPEEN

(11) **EP 1 712 914 A1**
(43) Date de publication de la demande: **18.10.2006**
(21) Numéro de dépôt: 05447079.4
(22) Date de dépôt: 14.04.2005
(51) Int. Cl.: G01N 33/544, G01N 33/558

(54) **Procédé in vitro pour la detection et l'identification simultanées d'antibiotiques de classes differentes et trousse de diagnostic correspondante**

(71) Demandeur: Unisensor S.A., 4031 Angleur (BE)
(72) Inventeur: Granier, Benoît, 4120 Rotheux-Rimiere (BE)
(74) Mandataire: Van Malderen, Michel

(57) **Abrégé**

La présente invention se rapporte à une trousse de diagnostic pour la détection ou la quantification simultanée et spécifique d'au moins deux analytes appartenant à des classes différentes, caractérisée en ce qu'elle comprend :
- d'une part, un seul mélange réactionnel comprenant au moins deux molécules biologiques différentes, capables chacune de reconnaître, respectivement, simultanément et spécifiquement, un analyte déterminé présent dans un échantillon pouvant contenir des analytes appartenant auxdites classes distinctes d'analytes ;
- et d'autre part, un système récupérateur auquel se trouvent fixés, en des emplacements distincts et connus dans l'espace, des ligands respectifs capables de récupérer spécifiquement, sélectivement et exclusivement chacune desdites molécules biologiques contenues dans ledit mélange réactionnel.

## Description

### Objet de l'invention

La présente invention se rapporte à un procédé consistant à faire réagir simultanément en un seul mélange réactionnel, un ensemble de diverses molécules biologiques de reconnaissance, capables de détecter spécifiquement plusieurs analytes distincts avec une sensibilité élevée et de déterminer la classe d'analytes à laquelle appartient chacun des composés détectés, sans que le principe de reconnaissance spécifique d'une classe d'analytes donnée ne puisse interférer sur le principe de reconnaissance spécifique d'une autre classe d'analytes.

L'invention concerne également la trousse de diagnostic spécialement conçue pour la mise en oeuvre du procédé.

### Arrière-plan technologique

Un principe fondamental qui gouverne les bonnes pratiques de surveillance et de contrôle de la chaîne alimentaire impose de réaliser des analyses de contrôle le plus en amont possible de la production pour permettre d'identifier et d'isoler le plus rapidement possible les denrées suspectées d'être contaminées.

En règle générale, lorsque l'on procède à des tests de dépistage souvent appelé "tests de screening", un échantillon détecté positif au cours d'une première analyse de contrôle est seulement supposé être effectivement positif et il doit subir obligatoirement un second test dit "de confirmation". *A contrario,* si un test initial de dépistage donne une réponse négative, elle est suffisante et aucune analyse ultérieure ne doit encore confirmer le résultat⁽¹⁾.

Une première conséquence de cette règle est que la première méthode de dépistage doit pouvoir couvrir la détection d'un maximum de composés. Les tests de screening ou de dépistage doivent donc préférentiellement et logiquement être des "tests multi-analytes".

Une seconde conséquence de cette règle est qu'il est important de connaître la classe à laquelle appartient le composé retrouvé dans un échantillon positif lors du test de screening de manière à pouvoir orienter directement vers la méthode de confirmation qui est en générale très particulière car il est recommandé d'isoler et d'identifier le composé concerné.

Une troisième conséquence de cette règle est qu'un test de screening ne peut pas donner de résultats de type "faux négatif" car ceux-ci échapperont dès lors à l'analyse à ce stade préliminaire et ne seront pas confirmés ultérieurement.

Actuellement, on connaît diverses méthodes pour la détection des antibiotiques :
- les tests microbiologiques qui mesurent le pouvoir inhibiteur d'un échantillon sur la croissance d'une souche bactérienne. Ce type de test demande un temps d'incubation relativement long (entre 3 et 16 heures) avant l'obtention du résultat. En général, ces tests (Delvotest® SP, BRT Test, Copan^{™}, Eclipse^{™}, Valio^{™}) ont la capacité de reconnaître plusieurs classes d'antibiotiques simultanément car les souches bactériennes utilisées sont souvent sensibles à plusieurs composés de classes différentes. Cependant, ce type de test ne permet pas d'identifier à quelle classe précise appartient le composé d'antibiotique testé ;
- les tests *in vitro* fonctionnent, dans le cas de la détection de petites molécules, selon un principe de compétition entre le composé à rechercher qui est présent dans l'échantillon et un compétiteur marqué qui a été volontairement introduit dans l'échantillon pour un seul et même site de reconnaissance qui peut être constitué soit par un récepteur, soit par un anticorps. Dans une formulation de type ELISA (*Enzyme Linked Immuno Sorbent Assay*) ou RIA (*Radio Immuno Assay*) / RRA (*Radio Receptor Assay*), le temps requis pour une analyse est de l'ordre de 2 à 6 heures. Certaines de ces méthodes, en particulier les méthodes RRA de laboratoire, permettent la détection simultanée de plusieurs composés de classes différentes. Cependant dans ce cas, la méthode ne permet pas d'identifier à quelle classe appartient le composé ayant donné une réponse positive ;
- les méthodes physico-chimiques, plus complexes, qui permettent d'isoler et d'identifier le composé recherché, sont, quant à elles, depuis peu, principalement des méthodes où un système de séparation chromatographique est couplé à un système de détection en spectrométrie de masse (GC/MS ou LC/MS). Ces méthodes demandent l'adaptation de protocoles particuliers pour chaque composé distinct à identifier. Tantôt, un seul composé ou une partie des composés d'une même classe peuvent être analysés simultanément, tantôt c'est l'ensemble des composés d'une même classe qui peuvent être détectés mais cela n'est jamais possible avec des composés de classes distinctes. En effet, le principe de séparation chromatographique est caractéristique des propriétés physico-chimiques d'un composé donné, ces dernières étant souvent différentes d'une classe à l'autre. Dans le cas où l'opérateur ne connaît pas le type de composé à identifier, il doit passer en revue autant de méthodes qu'il y a de classes de composés.

Depuis quelques années, on assiste également au développement de méthodes beaucoup plus rapides. Ces méthodes, dites "RAPID TESTS" sont utilisées pour effectuer un dépistage rapide sur un grand nombre d'échantillons. En général ces méthodes exploitent la reconnaissance des composés recherchés vis-à-vis d'une molécule biologique selon un principe de compétition. Pour rendre l'analyse rapide et pratique, ces types de tests fonctionnent à l'aide de dispositifs membranaires à flux latéral (Tetrasensor^{™}, SNAP®, Beta-STAR^{™}, ROSA). Ces tests se déclinent selon diverses classes d'antibiotiques mais aucun produit connu à ce jour ne permet de détecter en une seule opération des composés appartenant à des classes différentes.

S'il est raisonnable d'envisager et d'imposer au secteur agroalimentaire des analyses de dépistage primaire, le secteur concerné sera demandeur d'une analyse la plus complète possible et qui puisse de préférence identifier un maximum de composés suspects. Il est en effet plus pratique et économique de réaliser un seul test multiple à partir d'un seul échantillon plutôt que de devoir réaliser un test particulier pour chaque composé particulier. Cette pratique est lourde en temps, en gestion des échantillons et en coût. Elle constitue un frein à la bonne gestion et au contrôle efficace des denrées alimentaires.

Le contrôle rencontre donc à ce stade une limite importante à l'efficacité qui est caractérisée par l'absence de tests multi-analytes qui permettraient de détecter en une seule opération et en moins de 10 minutes par exemple, l'ensemble des composés de plusieurs classes d'analytes.

En particulier, le secteur agroalimentaire serait intéressé par un nouveau procédé permettant d'envisager en une seule opération l'analyse de composés appartenant à au moins deux classes différentes d'antibiotiques.

Le type d'antibiotique qui peut être administré à des animaux peut varier selon qu'il s'agit d'une application thérapeutique ou prophylactique, selon l'espèce animale, le germe à combattre, les pratiques vétérinaires, la législation en vigueur, les moyens disponibles ou encore les régions géographiques. Dans le cas de certains traitements particuliers, on utilise un mélange de médicaments. En règle générale, le praticien utilise des produits antibiotiques choisis parmi tous les composés commercialement disponibles selon son appréciation de la meilleure efficacité.

Les principales classes d'antibactériens et d'antibiotiques utilisés sont : les pénicillines et céphalosporines, les tétracyclines, les sulfamides, les aminoglycosides et aminocyclitols, les macrolides, les chloramphénicols ou autres peptides, ionophores, nitrofurannes, quinolones, carbadox, etc. Toutes ces classes regroupent un ensemble très vaste de composés chimiquement différents.

On pense que l'utilisation, parfois intensive, d'antibiotiques en médecine vétérinaire et en production agricole pourrait être à l'origine de l'émergence de souches bactériennes devenues résistantes aux antibiotiques. Pour préserver la santé humaine et légiférer en la matière, de nombreux pays (Union européenne, USA, Canada, etc.) ont établi des limites maximales autorisées (MRL) pour les résidus d'antibiotiques dans les denrées alimentaires⁽²⁾. Ces MRLs fixent en quelque sorte la limite entre un échantillon positif et un échantillon négatif, c'est-à-dire entre un échantillon refusé et un échantillon accepté.

Par ailleurs, la Décision de la Commission du 12 août 2002 a fixé les limites de performances minimales requises (LPMR) applicables aux méthodes d'analyse à utiliser pour l'examen des échantillons et a défini des critères communs pour l'interprétation des résultats⁽³⁾.

Il est entendu que seules les techniques d'analyse dont on peut démontrer, sur base de preuves identifiables, qu'elles sont validées et ont un taux de faux conformes inférieurs à 5% au niveau considéré doivent être appliquées aux fins de dépistage conformément à la Directive 96/23/CE.

En 1995, en moyenne un pour cent de tous les échantillons analysés par rapport à leur contenu en antibiotiques présentaient une teneur supérieure à la MRL et donnaient une réponse positive. Lorsque ces résultats positifs ont été confirmés, les produits le plus souvent rencontrés étaient des pénicillines et des tétracyclines⁽⁴⁾.

### Etat de la technique

Un système de reconnaissance des molécules de tétracycline est décrit dans la demande de brevet de la Demanderesse WO-A-03/048770 et intitulée "Procédé pour effectuer un diagnostic *in vitro* au moyen de mécanismes de régulation génétique et trousse de diagnostic correspondante". Cette méthode ne permet pas de détecter autre chose que des tétracyclines. Dans une formulation préférée, les réactifs sont constitués d'un récepteur tetR, isolé du mécanisme de régulation génétique d'*E*. *coli* pour la résistance aux tétracyclines, d'un anticorps capable de reconnaître le récepteur et d'une préparation de protéine-A conjuguée à des particules d'or colloïdales. D'autre part, le système récupérateur est un fragment d'ADN spécifique et biotinylé qui est immobilisable sur une molécule d'avidine fixée sur une membrane de nitrocellulose. C'est pendant l'incubation en présence de l'échantillon que le récepteur et le fragment d'ADN interagissent uniquement en l'absence de tétracycline. Dans ce cas, le fragment d'ADN forme un complexe avec le récepteur et un signal généré par les particules d'or fixées au récepteur apparaît. Dans le cas contraire le récepteur qui a reconnu préalablement une molécule de tétracycline n'est plus capable de se lier au fragment d'ADN et en conséquence aucun signal n'apparaît.

Un système de reconnaissance des β-lactames est décrit dans le brevet WO-A-99/67416 intitulé "Procédé pour la détermination d'antibiotiques à noyaux β-lactame dans un liquide biologique". Selon ce procédé, il n'est pas possible de détecter autre chose que des β-lactames. Dans une formulation préférée, les inventeurs décrivent d'une part un récepteur isolé de *Bacillus licheniformis,* purifié et couplé chimiquement à des molécules de biotine, auquel est associé une préparation d'anticorps anti-biotine conjugués à des particules d'or colloïdales, et d'autre part un conjugué formé d'une céphalosporine couplée à une immunoglobuline humaine et immobilisé sur une membrane de nitrocellulose. Dans le cas où l'échantillon ne contient pas de β-lactame, le récepteur se fixe à l'antibiotique immobilisé et un signal généré par les particules d'or fixées au récepteur-biotine, par l'intermédiaire d'un anticorps anti-biotine couplé à l'or, apparaît. Dans le cas contraire, si le récepteur est inhibé au cours d'une incubation préalable avec l'échantillon à tester, il n'est plus capable de se lier à l'antibiotique immobilisé et en conséquence aucun signal n'apparaît.

On connaît aussi un système de reconnaissance rapide des sulfamides décrit par R. Verheijen et *al.* ⁽⁵⁾ et intitulé "Development of a one step strip test for the detection of sulfadimidine residues". Ce système utilise un principe similaire à celui de la détection des β-lactames mentionné ci-dessus. D'une part, un anticorps spécifique des sulfadimidines est conjugué à des particules d'or colloïdales et c'est une sulfadimidine qui est conjuguée à de l'ovalbumine et immobilisée sur une membrane de nitrocellulose. Si l'échantillon contient des sulfadimidines reconnues par l'anticorps, celui-ci forme un complexe qui est incapable de rencontrer ultérieurement le composé immobilisé et en conséquence ne pourra pas s'y fixer. Dans le cas contraire en absence de sulfadimidine dans l'échantillon, l'anticorps conjugué pourra reconnaître le sulfadimidine immobilisée et un signal coloré apparaîtra. Cet article n'indique pas la spécificité de ce test.

Toutes les méthodes et procédés rapides en flux latéral, connus à ce jour, s'appliquent uniquement à la détection d'une seule et même classe de composés et il n'existe pas à ce jour de procédé permettant de détecter en une seule opération tous les composés appartenant à aux moins deux classes distinctes d'antibiotiques. Les raisons principales en sont une incompatibilité technique de réunir de manière indépendante et non-interférente, dans un seul et même procédé, tous les réactifs nécessaires à la détection de chacune des classes. La seconde difficulté réside ensuite dans la manière d'arriver à l'identification de la classe à laquelle appartient le composé détecté.

En résumé, les systèmes rapides connus pour la détection de petites molécules (MW < 2000) fonctionnent sur le principe de compétition qui demande l'utilisation de deux éléments particuliers que sont, d'une part, une molécule qui puisse reconnaître spécifiquement le composé à détecter, cette molécule étant en général un récepteur bactérien ou une immunoglobuline (anticorps), et d'autre part, un compétiteur pour le site de reconnaissance. Selon la méthode choisie, un des éléments est immobilisé sur un support et l'autre élément est marqué. Dans certains cas, c'est la molécule de reconnaissance qui est marquée et un analogue de l'analyte qui est immobilisé ; dans d'autres cas, c'est un analogue de l'analyte qui est marqué et c'est la molécule de reconnaissance qui est solidaire d'une fraction insoluble.

L'originalité du système de détection des tétracyclines se caractérise par le fait que le récepteur comprend deux sites de reconnaissance interdépendants et en conséquence, le compétiteur n'est pas un analogue de l'analyte recherché mais un fragment d'ADN capable de se fixer au second site de reconnaissance du même récepteur.

Dans toutes les méthodes dites "RAPID TESTS", l'appréciation est faite en comparant l'intensité du signal obtenu à la zone "test", c'est-à-dire à l'endroit ou l'élément récupérateur du récepteur actif est immobilisé par rapport à l'intensité d'un autre signal qui est obtenu à la zone "contrôle", c'est-à-dire à l'endroit où d'autres réactifs (ou l'excédent des réactifs) sont récupérés. En général, lorsque l'intensité à la zone "test" est plus marquée qu'à la zone "contrôle", le résultat est négatif pour l'élément recherché.

Dans tous les cas relatifs au dosage des molécules de β-lactames, la molécule de reconnaissance est obtenue à partir de préparation de *Bacillus* et dans tous les cas, cette molécule est marquée après purification. Pour réaliser ce marquage, il y a lieu de procéder à une modification chimique de surface. Par exemple, elle peut être couplée chimiquement à une enzyme, comme c'est le cas avec le récepteur de *Bacillus stéarothermophillus* couplé à de la peroxydase (EP-A-0 593112 et US-A-5,434,053 de Giest Brocades) ou à une biotine, comme c'est le cas avec le récepteur de *Bacillus licheniformis* couplé à une biotine (WO-A-99/67416 et US-A-6,524,804 de U.C.B. S.A.) ou encore avec le récepteur de *Bacillus stéarothermophillus* directement conjugué à de l'or colloïdal (US-A-6,475,805 de Charm Inc.). A l'exception de la méthode Tetrasensor^{™}, dans les autres cas connus à ce jour, il est nécessaire de modifier chimiquement la molécule de reconnaissance pour en obtenir un complexe coloré. En conséquence, il faut la purifier et en modifier certains des substituants de surface, au risque d'en modifier une propriété importante de fonctionnalité ou de stabilité.

Dans les autres cas connus à ce jour, il est en conséquence généralement impossible de travailler soit avec des extraits bruts non purifiés soit avec des récepteurs qui ne seraient pas modifiés chimiquement.

Par ailleurs, le type de marquage qui est exploité dans le dosage "tétracycline" n'est pas compatible avec le dosage des β-lactames car, dans celui-ci, c'est une immunoglobuline qui est immobilisée à la zone "contrôle" dans tous les cas renseignés. Dans le cas du document WO-99/67416, c'est aussi une immunoglobuline qui sert de protéine d'ancrage pour immobiliser le compétiteur à la zone "test". L'utilisation de la protéine-A, comme dans le dosage des tétracyclines, provoquerait inévitablement un marquage non spécifique sur une ou sur les deux lignes de capture nécessaires à la détection des β-lactames.

Dans un autre ordre d'idée, lorsque l'on doit recourir à l'utilisation de l'ensemble avidine-biotine pour faciliter soit la fixation sur le support, soit le marquage, l'ensemble avidine-biotine ne peut être utilisé dans une méthode donnée que pour une seule reconnaissance spécifique. En effet si une avidine constitue un point d'ancrage, toutes les molécules biotinylées pourront venir s'y fixer. Dans un tel cas, il est notamment irréalisable d'envisager un test pour la détection commune des β-lactames et des tétracyclines qui serait basé par exemple sur la réunion des deux principes de dosage décrits dans le document WO-A-99/67416 et dans le document WO-A-03/048770. En effet, dans le premier cas le récepteur est biotinylé pour ensuite être marqué à l'aide d'antibiotine-or et dans le second cas de l'avidine est immobilisée pour récupérer un fragment d'ADN biotinylé. La réunion de ces deux principes conduirait encore à un autre conflit entre les deux systèmes indépendants utilisant les couples avidine-biotine. Dans une réunion des deux systèmes existants, le récepteur biotinylé du système β-lactame se fixerait dans l'avidine nécessaire à récupérer l'ADN du système tétracycline et par voie de conséquence occasionnerait un marquage non spécifique indépendamment de la teneur en tétracycline dans l'échantillon. De plus la présence d'antibiotine-or dans la phase mobile, interférerait avec l'ADN biotinylé empêchant celui-ci de rencontrer l'avidine nécessaire à sa capture ce qui aboutirait encore à un autre conflit.

Dans un troisième ordre d'idée, la réunion de deux systèmes indépendants de reconnaissance demanderait un système de lecture et d'interprétation du résultat assez complexe où chacun des marqueurs doit s'apprécier par l'intensité relative à une référence interne. Il y aurait en conséquence deux bandes "test" ("beta" et "tetra") et deux bandes "contrôle". Cette vision ne rend l'analyse ni simple, ni pratique. Dans le cadre d'une réunion des deux tests, il vaudrait mieux apprécier chacune des intensités des tests par comparaison entre elles. Dans une telle pratique, la zone "test" pour l'analyte n°1 deviendrait la zone "contrôle" pour l'analyte n°2 et vice versa. Dans le cas ou les deux classes d'analytes concernées seraient présentes en même temps dans un même échantillon, cette approche générerait pas ou peu de signal. C'est rarement le cas mais s'il se présentait, pour éviter une interprétation difficile dans le cas d'une double contamination par des β-lactames et tétracyclines, il convient de préconiser l'ajout d'une référence unique sous la forme d'une seule ligne contrôle permettant plus aisément d'apprécier l'intensité relative des deux signaux tests. Il est évident que cette ligne unique de contrôle deviendrait nécessaire dans le cas où plus de deux tests seraient intégrés dans un même procédé.

Dans un quatrième ordre d'idée, les matériaux choisis expressément et préférentiellement pour constituer le dispositif de tigette de la trousse de diagnostic doivent être compatibles pour l'analyse simultanée des deux classes d'analytes. En effet dans une modalité d'exécution préférée décrite dans WO99/67416, il est recommandé d'utiliser la membrane de type "Leukosorb®" qui n'est pas compatible avec l'approche que nous préconisons pour le test des tétracyclines et ne conviendrait pas pour un dosage multi-analyte. En effet, très étrangement, dans une formulation ou les deux récepteurs sont marqués à l'aide de leur anticorps respectif, il est impossible d'obtenir un point de coupure net par ajout de β-lactames lorsque les réactifs rencontrent la membrane Leukosorb® avant d'atteindre le point de capture. Cette membrane, dont l'efficacité a été démontrée pour la purification du lait et l'utilisation en flux latéral, est la cause de signaux de reconnaissance non spécifiques lorsque le marquage est réalisé à l'aide d'anticorps et de protéine-A conjuguée à de l'or colloïdal.

Dans un cinquième ordre d'idée, le choix des récepteurs impliqués dans la reconnaissance de l'ensemble des composés de chacune des classes est important. Ce choix n'est pas uniquement déterminé par les performances de reconnaissance des composés mais également par des critères de stabilité, de structure, de réactivité antigénique, de composition en acides aminés, etc.

En conclusion, sur base des procédés connus, il est *a priori* impossible de rassembler dans un seul et même procédé au moins deux procédés indépendants, par exemple pour la reconnaissance par des récepteurs aux β-lactames et aux tétracyclines, sans envisager des modifications substantielles autorisant à réunir tous les éléments nécessaires, en s'assurant que le système de reconnaissance d'un composé n'interfère pas avec le système de reconnaissance d'un autre composé.

### Buts de l'invention

La présente invention vise à fournir un nouveau procédé de diagnostic permettant de détecter simultanément un ensemble -en principe illimité- de composés pouvant appartenir à au moins deux classes distinctes d'analytes et de caractériser la classe à laquelle appartient effectivement un composé détecté.

En particulier, l'invention vise à démontrer la compatibilité technique et pratique de réunir en un seul et même procédé au moins deux mécanismes de détection sans que le fonctionnement de l'un d'entre eux ne puisse interférer avec le fonctionnement de l'autre mécanisme.

Un but complémentaire de l'invention est de démontrer la faisabilité technique d'un dosage multi-analytes qui peut s'opérer rapidement, par exemple en moins de 10 minutes et en une seule et même étape d'analyse à l'aide d'un seul et même échantillon.

Un but complémentaire de l'invention est de fournir un procédé et une trousse de diagnostic *in vitro* pour la détection et le dosage simultanés d'au moins deux classes d'analytes.

### Principaux éléments caractéristiques de l'invention

Un premier objet de la présente invention, énoncé dans la revendication 1, se rapporte à une trousse de diagnostic pour la détection ou la quantification simultanée et spécifique d'au moins deux analytes appartenant à des classes différentes, caractérisée en ce qu'elle comprend :
- d'une part, un seul mélange réactionnel, de préférence sous forme d'une solution ou d'un lyophilisat, comprenant au moins deux molécules biologiques différentes, capables chacune de reconnaître, respectivement, simultanément et spécifiquement, un analyte déterminé présent dans un échantillon pouvant contenir des analytes appartenant auxdites classes distinctes d'analytes ;
- et d'autre part, un système récupérateur sous la forme d'un support solide unique auquel se trouvent fixés, en des emplacements distincts et connus dans l'espace, des ligands respectifs capables de récupérer spécifiquement, sélectivement et exclusivement chacune desdites molécules biologiques contenues dans ledit mélange réactionnel, de manière à identifier par l'emplacement de la récupération sur ledit support, le type de classe à laquelle appartient chacun des analytes présents dans l'échantillon.

Des modalités préférées de l'invention sont décrites dans les revendications secondaires 2 à 18.

Un second objet de l'invention concerne un procédé de mise en oeuvre d'une trousse de diagnostic selon l'une quelconque des revendications 1 à 18, caractérisé par les étapes suivantes :
- on met en contact le mélange réactionnel précité avec un échantillon à caractériser pour obtenir une solution qu'on laisse incuber à 50°C pendant 3 minutes ;
- on plonge une tigette portant le système récupérateur précité dans la solution obtenue et on laisse incuber pendant 3 minutes ;
- on interprète quantitativement le résultat sur la tigette au moyen d'un lecteur optique de tigette.

Dans les formes d'exécution préférées de la présente invention, on préconise d'éviter d'une part la purification et d'autre part la modification chimique du récepteur en ayant recours à des anticorps anti-récepteur qui seraient marqués soit directement à l'or colloïdal soit préférentiellement à l'aide de protéine-A couplée à l'or colloïdal. D'autre part, dans une idée de multiplicité de détection, la protéine-A marquée à l'or colloïdal servirait de marqueur générique pour tous les anticorps introduits dans la solution.

Afin de préserver au maximum les fonctionnalités des récepteurs et anticorps utilisés dans la présente invention, un principe adopté est qu'aucun marquage par modification chimique n'a lieu. En conséquence la présente invention exploite les récepteurs bactériens dans leur état le plus naturel possible. Ces récepteurs sont marqués à l'aide d'anticorps qui seront eux- mêmes reconnus par une proteine-A conjuguée à de l'or colloïdal. C'est donc exclusivement la protéine de marquage qui sera couplée à l'or mais pas les molécules sensibles pour la reconnaissance des analytes concernés.

Dans cette approche, tous les groupements fonctionnels des récepteurs sont donc préservés. Cet avantage prend toute son importance lorsqu'il s'agit d'exploiter l'activité de récepteurs dont le mécanisme de reconnaissance dépend de certaines chaînes latérales comme les résidus lysines par exemple. C'est d'autant plus important que lorsqu'une modification chimique à lieu, dans la plupart des cas renseignés, la modification implique les résidus NH₃⁺ des chaînes latérales des lysines. En conclusion, un couplage chimique sur les lysines peut altérer des lysines du site actif et rendre ainsi la préparation partiellement inactive.

Le marquage par l'intermédiaire d'anticorps présente la souplesse d'un marquage non figé. En effet, un marquage chimique implique une liaison covalente et irréversible alors qu'une fixation d'anticorps est réversible et sujette à un équilibre qui peut être déplacé selon les forces en action. Un autre avantage est de pouvoir moduler l'étape de marquage dans un deuxième temps. En effet, dans une deuxième formulation de la présente invention, c'est après la phase de reconnaissance de l'analyte par le récepteur, que ce soit l'analyte libre de l'échantillon ou l'analyte immobilisé pour la récupération, que l'anticorps reconnaît le récepteur.

Pour diverses raisons économiques ou de stabilité, mais aussi pour une parfaite intégration dans un test multi-analytes, la présente invention préconise préférentiellement l'utilisation d'un récepteur des β-lactames isolé à partir de *Staphylococcus aureus*⁽⁶⁾.

En effet, étant originaire de la souche bactérienne la plus performante à l'égard de la lutte contre les antibiotiques et les β-lactames en particulier, ce récepteur utilisé *in vitro* a démontré des capacités exceptionnelles de reconnaissance de l'ensemble de composés des β-lactames, tant pour les pénicillines que pour les céphalosporines (voir réf.⁽⁶⁾ et résultats repris en exemple).

Un autre avantage de ce récepteur est d'avoir un point isoélectrique (pI) basique. Ceci offre la possibilité de pouvoir le purifier très facilement à partir d'un extrait brut de cellules pour en obtenir des anticorps.

Par ailleurs, notre choix a également été déterminé par l'observation d'une très bonne réponse immunitaire chez le lapin, très spécifique et n'occasionnant aucune réponse non spécifique avec le reste de l'ensemble des réactifs impliqués dans le dosage multi-analytes.

Selon la présente invention, on est parvenu à formuler un procédé dans lequel il est possible de réunir dans une seule et même opération tous les éléments nécessaires à la détection commune de plusieurs composés de classes différentes en utilisant des mécanismes de reconnaissance totalement différents.

La présente invention offre l'avantage supplémentaire de proposer une formulation d'un test multi-analytes fiable et économique. En effet, il n'est pas nécessaire de purifier les récepteurs, ni les préparations d'anticorps et de plus les récepteurs ne sont pas modifiés chimiquement, ce qui devrait préserver l'intégralité de leur réactivité ainsi que leur stabilité.

### Brève description des figures

La figure 1 représente schématiquement le positionnement des éléments récupérateurs selon l'invention sur un support solide de nitrocellulose dans le cas du dosage simultané de tétracyclines et de β-lactames à l'aide de récepteurs spécifiques, une zone de contrôle fixe étant également prévue.

La figure 2 représente schématiquement le positionnement des éléments récupérateurs selon l'invention sur un support solide de nitrocellulose dans le cas du dosage simultané de tétracyclines, de β-lactames et de sulfamides, une zone de contrôle fixe étant également prévue.

### Description de modes d'exécution préférés de l'invention

### Exemple N°1 : Dosage simultané des tétracyclines et des β-lactames à l'aide de récepteurs spécifiques

Le procédé utilise un mélange réactionnel contenant les deux récepteurs respectifs des β-lactamines et tétracyclines ainsi que leurs réactifs et un élément récupérateur auquel se trouvent immobilisés, à des endroits précis et distincts, des ligands spécifiques de ces récepteurs.

### Préparation du mélange réactionnel

Pour la détection des β-lactames, le mélange réactionnel contient le récepteur spécifique de reconnaissance des β-lactames, son anticorps spécifique et une préparation de protéine-A conjuguée à de l'or colloïdal. Pour la détection des tétracyclines, le mélange réactionnel contient le récepteur des tétracyclines, son anticorps spécifique, un fragment d'ADN spécifique conjugué à de la biotine et une préparation de protéine-A conjuguée à de l'or colloïdal.

Le récepteur β-lactame est obtenu selon la méthode décrite par Golemi-Kotra et al. ⁽⁶⁾. Le récepteur non purifié est filtré sur membrane Millex HV (Millipore, Inc, USA) avant d'être conservé à 4°C en présence de glycérol 50% v/v. Le récepteur tétracycline est obtenu selon la méthode décrite dans la demande de brevet WO-A-03/048770.

Les deux préparations d'anticorps sont obtenues selon la méthode décrite dans Kachab et al. ⁽⁷⁾. Ces préparations d'anticorps ont été obtenues par injection d'une préparation de récepteur purifié jusqu'à homogénéité protéique selon Golemi-Kotra et al.⁽⁶⁾ pour le récepteur aux β-lactames et selon la demande WO-A-03/048770 pour le récepteur aux tétracyclines. Les deux préparations d'anticorps sont utilisées préférentiellement non purifiées ce qui signifie que c'est du sérum non purifié qui est directement ajouté au mélange réactionnel. Selon une seconde modalité préférée, les anticorps spécifiques, de type polyclonal ou monoclonal, sont purifiés selon les méthodes connues de l'homme de métier pour ensuite être couplés soit directement soit indirectement à des particules d'or colloïdal selon la méthode de Frens⁽⁸⁾.

Le fragment d'ADN est obtenu chez Eurogentec SA, Belgique, selon la séquence et la préparation reprises dans la demande de brevet WO-A-03/048770. Dans ce cas précis, le fragment d'ADN n'est pas immobilisé à un point de capture sur une membrane de nitrocellulose mais il est directement intégré au mélange réactionnel. Ce fragment sera récupéré *in testo* à l'aide de son extrémité biotinylée sur de l'avidine immobilisée à un point de capture (cf. ci-après).

L'ensemble des réactifs nécessaires sont introduits sans aucune modification chimique ni substitution de manière à préserver l'intégralité des propriétés d'activité et de stabilité. Ces molécules peuvent être purifiées ou non purifiées ce qui présente un avantage économique évident.

Dans l'exemple précis, le mélange réactionnel contient :
- 5 parts de récepteur β-lactame (RSA) à une concentration de 360 nM ;
- 5 parts de récepteur tétracycline (TetR) à une concentration de 4,2 µM ;
- 1 part de sérum anti-RSA diluée 4 x en tampon NaPi 50 mM, NaCl 150 mM, pH 7,8 ;
- 1 part de sérum anti-TetR diluée 4 x en tampon NaPi 50 mM, NaCl 150 mM, pH 7,8 ;
- 1 part d'acide nucléique à une concentration de 25 µM en NaCl 690 mM ;
- 15 parts de protéine-A Or de 40 nm à une DO=10 à 520nm ;
- 22 parts de tampon Hepes 120 mM, pH 8, BSA 2%, Dextran 1%, sucrose 5%.
Ce mélange est soit préparé extemporanément soit lyophilisé pendant 20 heures.

### Le système récupérateur.

Le système récupérateur est constitué d'une membrane de nitrocellulose sur laquelle se trouvent fixés, à des endroits distincts, les éléments capables de former un complexe avec les molécules de reconnaissance du mélange réactionnel. Ces éléments sont :
- pour le signal dit "beta" (pour β-lactame), un antibiotique, de préférence de type pénicilline ou céphalosporine, immobilisé sur une molécule ne présentant aucune réactivité particulière avec la protéine-A ou la biotine ou encore l'avidine. Dans un mode d'exécution préféré de la présente invention, on utilise une ampicilline immobilisée sur une β-lactoglobuline ;
- pour le signal dit "tetra" (pour tétracycline), une avidine capable de reconnaître un fragment d'ADN présentant à une extrémité une molécule de biotine. De préférence, on utilisera une avidine d'oeuf.

Les zones de capture "beta" et "tetra" peuvent être localisées successivement l'une avant l'autre, ou inversement, sans préférence particulière pour autant qu'elle soit disposées à des emplacements spatialement distincts mais connus. C'est en effet cette position distincte qui permettra de repérer le type d'antibiotique présent dans la solution d'analyse. Si les deux systèmes de capture étaient réunis en un seul endroit, le dosage n'en serait pas altéré mais il ne serait pas possible de déterminer quel type de composé est effectivement présent.

### Préparation de β-lactoglobuline conjuguée à une ampicilline

La β-lactoglobuline est choisie car c'est une protéine du lait qui contient beaucoup de résidus lysine (10%) et la structure tridimensionnelle indique que la plupart des lysines présentent leur extrémité NH₂ vers l'extérieur de la protéine (Pubmed, structure, 1GXA).

100 mg de β-lactoglobuline et 15 mg de 2-iminothiolane sont incubés en tampon NaPi 100mM, pH 8,5 dans un volume réactionnel de 4 ml pendant 60 minutes à 25°C. Le mélange est ensuite déposé sur une colonne PD10 (Amersham-Biosciences, UK) préalablement équilibrée en PBS pH 7, EDTA 5mM et éluée dans ce même tampon. Les fractions protéiques sont rassemblées selon les méthodes bien connues.

D'autre part, 2 ml de DMSO contenant 100 mg de SICC sont incubés avec 2 ml d'une solution de 50 mg/ml d'ampicilline sodique en tampon NaPi 25 mM, pH 8, pendant 60 minutes à 25°C, avant d'être incubé en présence de 50 mg de solution protéique pendant 4 heures à 4°C. La solution est enfin dialysée deux fois 6 heures contre 100 volumes de tampon NaPi 25 mM, pH 7,5.

### Préparation d'une solution d'avidine neutralisée

Une solution de 5 mg/ml d'avidine d'oeuf, disponible chez Pierce Inc, USA, est préparée en tampon NaPi 50 mM, pH 7,5.

### Préparation d'une solution contrôle de BSA-Or.

La présente invention préconise l'utilisation d'une ligne de contrôle ayant une intensité constante et visible avant et après le développement du test. Cette ligne est de préférence de couleur similaire à la couleur développée aux lignes "tests" et est synthétisée comme suit.

A une préparation de 27 ml d'or colloïdal de 40 nm, obtenue selon la méthode de Frens⁽⁸⁾ et dont la densité optique DO_{lambda max} est de 3, sont ajouté 3 ml d'une solution de BSA (Sigma) à 10% en tampon borate 10 mM, pH 6,5. Le mélange est incubé pendant 60 minutes à 20°C avant d'être centrifugé 45 minutes à 10.000 rpm dans un rotor SS34 (Sorvall). Le culot est ensuite récupéré dans du tampon NaPi 50 mM, NaCl 150 mM de manière à obtenir une DO_{lambda max} de 45. La solution qui sera déposée sur la membrane de nitrocellulose est encore diluée 15 fois dans le même tampon pour obtenir une DO finale de 3.

### Technique immunochromatographique.

La technique immunochromatographique est connue et décrite dans la littérature (Developing Immunochromatographic Test Strips : A short Guide, Millipore, Lit. No. TB500 Printed in USA 11/96, 96-204). Dans la présente invention, les préparations obtenues ci-dessus sont déposées en des endroits précis et distincts sur une membrane en nitrocellulose et de préférence successivement l'un derrière l'autre en se référant au sens de migration du liquide. La membrane en nitrocellulose est ensuite placée en contact à une extrémité avec une membrane par exemple de type 142 disponible chez Ahlstrom, Inc., USA ou de type GFDVA disponible chez Whatman, UK, mais pas de type Leukosorb® disponible chez Pall, UK et à l'autre extrémité par un papier absorbant quelconque par exemple de type 17CHR disponible chez Whatman, UK.

### Dépôt des éléments récupérateurs sur le support de nitrocellulose

C'est le positionnement des éléments récupérateurs qui permettront d'identifier le type de contamination rencontrée dans l'échantillon. Les solutions sont déposées à l'aide d'un "dispenseur" Biodot (UK) de type Quanti-3000, à un débit de 1 ml/min.

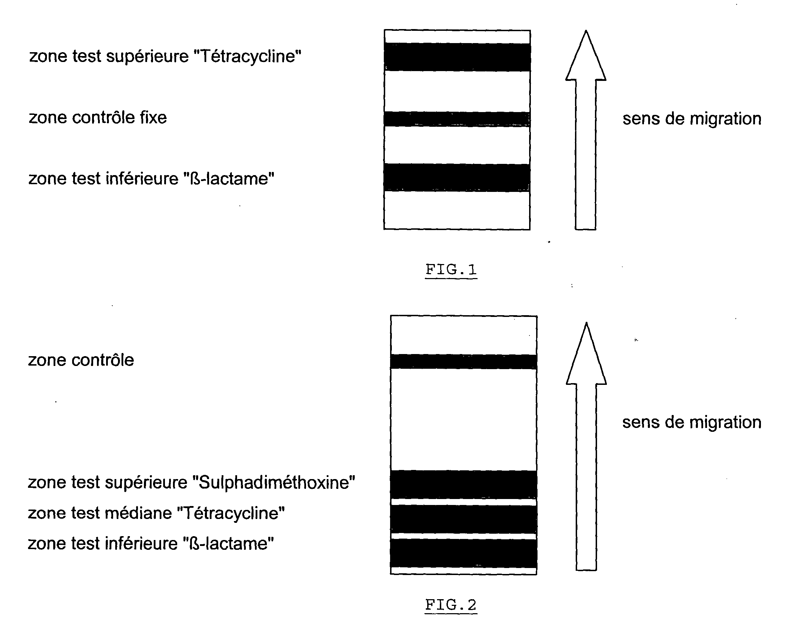

Dans le cas d'une détection de deux paramètres, c'est de part et d'autre de la ligne contrôle que les préparations de β-lactoglobuline et d'avidine seront déposées. Par exemple le conjugué β-lactoglobuline préparé ci avant, sera déposé en dessous de la ligne contrôle et la solution d'avidine neutralisée sera déposée au-dessus de la ligne de contrôle (figure 1). En conséquence, la présence de composé β-lactames se caractérisera par une absence de marquage sur la β-lactoglobuline, c'est-à-dire à la ligne test située en dessous de la ligne de contrôle et la présence de composés tétracyclines se caractérisera par une absence de marquage à la ligne test située au-dessus de la ligne contrôle. Dans le cas où les deux composés sont présents en quantité suffisante aucun des deux signaux n'apparaîtra, de part et d'autre de la ligne de contrôle (voir figure 1).

### Dosage simultané des β-lactames et des tétracyclines dans un échantillon de lait

200 µL de lait froid, sont incubés à 50°C en présence de 50 µl des réactifs préparés et/ou lyophilisés comme ci-dessus. Après 3 minutes d'incubation à 50°C, l'élément récupérateur décrit ci-dessus est plongée dans la solution. L'interprétation finale est faite après 3 minutes d'incubation à l'aide d'un lecteur optique de tigette disponible chez Matest (Allemagne).

Les résultats sont repris dans le tableau 1. Le procédé permet de considérer comme positif un échantillon de lait contenant 4 ppb en Ampicilline et/ou 75 ppb en Tétracycline.

Le tableau 2 résume les limites de détection obtenues par le présent procédé pour les divers composés de β-lactames et de tétracyclines renseignés. En règle générale lorsque le rapport des intensités du signal de test concerné par rapport au signal de contrôle est égal ou inférieur à 1, l'échantillon est considéré comme positif pour le paramètre (composé) concerné.

**Tableau 1**

| Unités et rapports des intensités mesurées aux points de capture | | | | | |
|---|---|---|---|---|---|
| ***Concentration antibiotique (ppb)*** | ***Intensité zone B*** (*) | ***Rapport B* / *C*** | ***Intensité zone T*** | ***Rapport T* / *C*** | ***Intensité zone contrôle*** |
| Ampi 0 + Tetra 0 | 520 | 2.5 | 410 | 2.0 | 208 |
| Ampi 1 | 380 | 1.9 | 380 | 1.9 | 199 |
| Ampi 2 | 250 | 1.2 | 405 | 1.9 | 211 |
| Ampi 3 | 155 | 0.8 | 408 | 2.0 | 205 |
| Ampi 4 | 105 | 0.5 | 386 | 1.9 | 201 |
| Ampi 5 | 55 | 0.3 | 397 | 2.0 | 197 |
| Ampi 10 | 25 | 0.1 | 414 | 2.1 | 200 |
| Ampi 0 + Tetra 25 | 505 | 2.5 | 340 | 1.7 | 206 |
| Ampi 0 + Tetra 50 | 521 | 2.5 | 288 | 1.4 | 206 |
| Ampi 0 + Tetra 75 | 523 | 2.6 | 162 | 0.8 | 203 |
| Ampi 0 + Tetra 100 | 508 | 2.6 | 55 | 0.3 | 199 |
| Ampi 4 + Tetra 75 | 122 | 0.6 | 165 | 0.8 | 202 |

| | | | | | |
|---|---|---|---|---|---|
| (*) B : beta ; T :tetra ; C : contrôle | | | | | |

**Tableau 2**

| Limite de détection | | | | |
|---|---|---|---|---|
| ***Antibiotique*** | ***MRL*/*UE (ppb)*** | ***Safe Level*/*US (ppb)*** | ***Concentration (ppb)*** | ***Rapport d'intensité paramètre* / *zone contrôle*** |
| Benzylpénicilline | 4 | 5 | 2 | 0.4 |
| Ampicilline | 4 | 10 | 4 | 0.4 |
| Amoxicilline | 4 | 10 | 4 | 0.4 |
| Cloxacycline | 30 | 10 | 10 | 0.5 |
| Céphapyrine | 60 | 20 | 8 | 0.3 |
| Ceftiofur | 100 | 50 | 10 | 0.5 |
| Tétracycline | 100 | 300 | 75 | 0.8 |
| Oxytétracycline | 100 | 300 | 50 | 0.8 |
| Chlortétracycline | 100 | 300 | 50 | 0.8 |
| Doxycycline | 100 | 300 | 20 | 0.8 |

### Exemple N°2 : Dosage simultané des tétracyclines, β-lactames et sulfamides

Dans cette forme d'exécution préférée, le procédé intègre en plus des deux récepteurs des tétracyclines et β-lactames, un anticorps spécifique pour la reconnaissance des sulfadiméthoxines.

### Préparation du mélange réactionnel

Au mélange renseigné ci-dessus, est intégré en outre un anticorps anti-sulphadiméthoxine selon la préparation suivante :
- 5 parts de récepteur β-lactame (RSA) à une concentration de 360 nM ;
- 5 parts de récepteur tétracycline (TetR) à une concentration de 4,2 µM ;
- 1 part de sérum anti-RSA diluée 4 x en tampon NaPi 50 mM, NaCl 150 mM, pH, 7,8 ;
- 1 part de sérum anti-TetR diluée 4 x dans ce même tampon ;
- 1 part de sérum anti-sulphadiméthoxine diluée 2 x dans ce même tampon ;
- 1 part d'acide nucléique à une concentration de 50 µM ;
- 20 parts de protéine-A Or de 40 nm à une DO=10 à 520nm ;
- 16 parts de tampon Hepes 120 mM, pH 8, BSA 2%, Dextran 1%, sucrose 5%.
Ce mélange est soit préparé extemporanément soit lyophilisé pendant 20 heures.

### Le système récupérateur.

Le système récupérateur est semblable à celui de l'exemple 1, dans lequel une quatrième ligne spécifique pour la récupération des molécules d'anticorps anti-sulphadiméthoxine est intégrée (3^{ème} ligne de test). Les autres zones de capture sont identiques mais positionnées autrement selon la figure 2.

### Préparation d'une BSA conjuguée à une sulphadiméthoxine.

La préparation est réalisée en suivant le protocole décrit par Dixon-Holland et Katz⁽⁹⁾. 100 mg de sulfadiméthoxine et 200 mg de BSA solubilisés dans 25 ml d'un mélange, contenant 2 parts de tampon phosphate 50 mM pH 7,2 et 1 part de dioxane, sont incubés en présence de 120 µL de 25% glutaraldéhyde sous agitation pendant 3 heures à 25°C. La solution est ensuite dialysée pendant 6 jours à 4°C contre 100 volumes de tampon NaPi 50 mM, pH 7,2 avec changement de tampon toutes les 12 heures.

### Dépôt des éléments récupérateurs sur le support de nitrocellulose

Dans le cas d'une détection de trois paramètres, les lignes de capture sont disposées en des endroits précis et distincts sur une membrane en nitrocellulose et de préférence successivement l'une dernière l'autre en se référant au sens de migration du liquide. Selon une modalité préférée, les zones de capture β-lactame, tétracycline, sulphadiméthoxine et la zone contrôle sont disposées respectivement à un premier, à un deuxième, à un troisième et enfin à un quatrième niveau en se référant au sens de migration du liquide (figure 2). Une seule zone de contrôle sert de référence pour les trois marqueurs. Une formulation différente, similaire à l'exemple précédent, serait d'intégrer deux zones de contrôle, c'est-à-dire une zone de contrôle entre chacune des zones de test.

### Dosage simultané des β-lactames, des tétracyclines et des sulphadiméthoxines dans un échantillon de lait

200 µL de lait froid sont incubés à 50°C en présence de 50 µl des réactifs préparés et/ou lyophilisés comme ci dessus. Après 3 minutes d'incubation à 50°C, l'élément récupérateur décrit ci-dessus est plongée dans la solution. L'interprétation finale est faite après 3 minutes d'incubation à l'aide d'un lecteur optique de tigette disponible chez Matest (Allemagne).

Les résultats sont repris dans le tableau 3. Le procédé permet de considérer positif un échantillon de lait contenant 4 ppb en ampicilline et/ou 75 ppb en tétracycline et 100 ppb en sulphadiméthoxine.

Le tableau 4 résume les limites de détection obtenues par le procédé de l'invention pour les divers composés de β-lactames, de tétracyclines et de sulphadiméthoxines renseignés. En règle générale, lorsque le rapport des intensités du signal de test concerné par rapport au signal de contrôle est égal ou inférieur à 1, l'échantillon est considéré comme positif pour le paramètre (composé) concerné.

**Tableau 3**

| Rapports des intensités mesurées | | | | | |
|---|---|---|---|---|---|
| ***Concentration antibiotique (ppb)*** | | | ***Rapport des intensités de mesure*** | | |
| ***A* (*)** | ***T*** | ***S*** | ***B*/*C*** | ***T*/*C*** | ***S*/*C*** |
| 0 | 0 | 0 | 2.5 | 2.0 | 1.9 |
| 1 | 0 | 0 | 1.9 | 1.9 | 1.7 |
| 2 | 0 | 0 | 1.2 | 1.9 | 2.1 |
| ***3*** | 0 | 0 | ***0.8*** | 2.0 | 2.2 |
| ***4*** | 0 | 0 | ***0.5*** | 1.9 | 2.0 |
| ***5*** | 0 | 0 | ***0.3*** | 2.0 | 2.0 |
| 10 | 0 | 0 | ***0.1*** | 2.1 | 1.9 |
| 0 | ***25*** | 0 | 2.5 | ***1.7*** | 1.9 |
| 0 | ***50*** | 0 | 2.5 | ***1.4*** | 2.2 |
| 0 | ***75*** | 0 | 2.6 | ***0.8*** | 2.0 |
| 0 | ***100*** | 0 | 2.6 | ***0.3*** | 1.8 |
| 0 | 0 | 50 | 2.4 | 1.9 | 1.3 |
| 0 | 0 | ***100*** | 2.2 | 1.9 | ***0.8*** |
| 0 | 0 | ***150*** | 1.9 | 2.1 | ***0.6*** |
| 0 | 0 | ***200*** | 2.4 | 2.1 | ***0.4*** |
| 4 | 75 | ***100*** | ***0.6*** | *0.8* | 0.7 |

| | | | | | |
|---|---|---|---|---|---|
| (*) A : ampicilline ; T : tétracycline ; S : sulfamide ; C : contrôle | | | | | |

**Tableau 4**

| Limite de détection | | |
|---|---|---|
| ***Antibiotique*** | ***Concentration limite détection (ppb)*** | ***Rapport Test* / *Contrôle*** |
| Benzylpenicilline | 2 | 0.4 |
| Ampicilline | 4 | 0.4 |
| Amoxicilline | 4 | 0.4 |
| Cloxacycline | 10 | 0.5 |
| Céphapyrine | 8 | 0.3 |
| Ceftiofur | 10 | 0.5 |
| Tétracycline | 75 | 0.8 |
| Oxytétracycline | 50 | 0.9 |
| Chlortétracycline | 50 | 0.7 |
| Doxycycline | 20 | 0.6 |
| Sulphadimétoxine | 100 | 0.8 |

### Références

(1) DIRECTIVE 96/23/CE DU CONSEIL, du 29 avril 1996, *relative aux mesures de contrôle à mettre en oeuvre à l'égard de certaines substances et leurs résidus dans les animaux vivants et leurs produits et abrogeant les Directives 85*/*358*/*CEE et 86*/*469*/*CEE et les Décisions 89*/*187*/*CEE et 91*/*664*/*CEE.*
(2) REGLEMENT (CEE) n° 2377/90 DU CONSEIL, du 26 juin 1990, *établissant une procédure communautaire pour la fixation* des *limites maximales de résidus de médicaments vétérinaires dans les aliments d'origine animale.*
(3) Off. J. Eur. Comm. 2002, L221/8 - 2002/657/CE.
(4) Hisao Oka, Chemical Analysis for antibiotics Used in Agriculture, by AOAC INTERNATIONAL, 1995, ISBN 0-935584-57-9.
(5) Ron Verheijen et al, Analyst 123 (1998), 2437-2441.
(6) Golemi-Kotra, D. et al, The Journal of Biological Chemistry, Vol. 278, n° 20 (May 2003), pp. 18419-18425.
(7) Kachab, E.H. et al, The Journal of Immunology Methods, Vol 147, n°1 (1992), pp. 33-41.
(8) Frens, G., Nature (London), Phys. Sci., 241 (1973), 20.
(9) Dixon-Holland, D.E. et Katz, S.E., (1988), J. Assoc. Off. Anal. Chem. (1988) 71 (6), 1137-40.

## Revendications

1. Trousse de diagnostic pour la détection ou la quantification simultanée et spécifique d'au moins deux analytes appartenant à des classes différentes, **caractérisée en ce qu'**elle comprend :
- d'une part, un seul mélange réactionnel comprenant au moins deux molécules biologiques différentes, capables chacune de reconnaître, respectivement, simultanément et spécifiquement, un analyte déterminé présent dans un échantillon pouvant contenir des analytes appartenant auxdites classes distinctes d'analytes ;
- et d'autre part, un système récupérateur sous la forme d'un support solide unique auquel se trouvent fixés, en des emplacements distincts et connus dans l'espace, des ligands respectifs capables de récupérer spécifiquement, sélectivement et exclusivement chacune desdites molécules biologiques contenues dans ledit mélange réactionnel, de manière à identifier par l'emplacement de la récupération sur ledit support, le type de classe à laquelle appartient chacun des analytes présents dans l'échantillon.

2. Trousse de diagnostic selon la revendication 1, **caractérisée en ce que** le support solide pour la fixation des ligands récupérateurs comprend une membrane ou un ensemble de membranes, une colonne contenant une couche ou un empilement de couches différentes ou encore une cavité ou un ensemble de cylindres emboîtés formant une cavité.

3. Trousse de diagnostic selon la revendication 1 ou 2, **caractérisée en ce que** les classes distinctes d'analytes comprennent des antibiotiques ou antibactériens, des hormones, des stéroïdes, des hydrocarbures ou encore sont des classes mixtes regroupant des composés appartenant à au moins deux de ces classes distinctes.

4. Trousse de diagnostic selon la revendication 3, **caractérisée en ce que** les classes des antibiotiques ou antibactériens sont celles des pénicillines, des céphalosporines, des tétracyclines, des sulfamides, des amynoglycosides, des aminocyclitols, des macrolides, des quinolones, des ionophores, des carbadox, des nitrofurannes ou des phénicols.

5. Trousse de diagnostic selon la revendication 1, **caractérisé en ce que** lesdites molécules biologiques de reconnaissance spécifique d'un analyte présent dans l'échantillon sont des anticorps ou des récepteurs biologiques, naturels, sauvages ou artificiellement modifiés génétiquement ou chimiquement, ou encore un ensemble varié de récepteurs et d'anticorps.

6. Trousse de diagnostic selon la revendication 1, **caractérisé en ce que** les ligands récupérateurs sont soit des analogues des analytes recherchés, soit des molécules capables de fixer spécifiquement lesdites molécules biologiques de reconnaissance.

7. Trousse de diagnostic selon la revendication 6, **caractérisée en ce que** les ligands récupérateurs sont des anticorps ou des protéines fixant lesdits anticorps de reconnaissance ou lesdits récepteurs ou encore des ligands desdits récepteurs.

8. Trousse de diagnostic selon la revendication 1, **caractérisée en ce que** lesdits ligands récupérateurs sont disposés soit de manière ordonnée par juxtaposition successive sous forme de matrice régulière ou d'alignement, parallèle ou perpendiculaire au sens de migration du liquide, soit de manière désordonnée, séparément à divers endroits ou rassemblés pour des besoins spécifiques en un seul et même emplacement.

9. Trousse de diagnostic pour le dosage simultané au moins de β-lactames et de tétracyclines selon la revendication 1, **caractérisée en ce que** :
- le mélange réactionnel contient un premier récepteur, spécifique de la reconnaissance des β-lactames et un anticorps spécifique de ce premier récepteur, un second récepteur, spécifique de la reconnaissance des tétracyclines, un anticorps spécifique de ce second récepteur, un élément possédant une extrémité biotinylée reconnu spécifiquement par le second récepteur et de la protéine-A conjuguée à des particules d'or colloïdales ;
- le système récupérateur comprend une membrane de nitrocellulose sur laquelle sont fixés en deux endroits connus et distincts appelés zones de test, respectivement un antibiotique de type pénicilline ou céphalosporine, de préférence une ampicilline immobilisée sur une β-lactoglobuline et une avidine capable de reconnaître l'élément présentant une extrémité biotinylée, de préférence une avidine d'oeuf.

10. Trousse de diagnostic pour le dosage simultané au moins de β-lactames et de tétracyclines selon la revendication 9, **caractérisée en ce que** le second récepteur, possédant un premier site de reconnaissance spécifique des tétracyclines, possède en outre un second site de reconnaissance spécifique pour un fragment d'acide nucléique possédant une extrémité biotinylée et se trouvant libre dans le milieu réactionnel précité.

11. Trousse de diagnostic selon la revendication 9 ou 10, **caractérisée en ce que** les récepteurs utilisés pour le dosage simultané des β-lactames et des tétracyclines sont respectivement les récepteurs BlaR et TetR isolés à partir de classes connues de microorganismes, de préférence respectivement à partir de *Staphylococcus aureus* pour BlaR et du plasmide pSC101 *d'E. coli 600* pour TetR.

12. Trousse de diagnostic pour le dosage simultané au moins de β-lactames et de sulfamides selon la revendication 1, **caractérisée en ce que** :
- le mélange réactionnel contient un récepteur, spécifique de la reconnaissance des β-lactames et un anticorps spécifique de ce premier récepteur, un second anticorps spécifique de la reconnaissance des sulfamides ainsi que de la protéine-A conjuguée à des particules d'or colloïdales ;
- le système récupérateur comprend une membrane de nitrocellulose sur laquelle sont fixés en deux endroits connus et distincts appelés zones de test, respectivement un antibiotique de type pénicilline ou céphalosporine, de préférence une ampicilline immobilisée sur une β-lactoglobuline et un antibactérien de type sulfamide, de préférence une sulfadiméthoxine immobilisée sur une albumine bovine.

13. Trousse de diagnostic selon l'une quelconque des revendications 9 à 12, **caractérisée en ce que** les anticorps anti-récepteurs spécifiques ajoutés au mélange réactionnel sont des anticorps soit monoclonaux, soit polyclonaux, modifiés ou non modifiés chimiquement ou par recombinaison, purifiés ou non purifiés, couplés ou non couplés à des particules d'or colloïdales.

14. Trousse de diagnostic selon l'une quelconque des revendications 9 à 13, **caractérisée en ce que** la membrane de nitrocellulose, déposée sur un support tigette, est en contact à une première extrémité avec une membrane et à une seconde extrémité avec un papier absorbant et présente les deux zones de test distinctes l'une derrière l'autre dans le sens de migration du liquide, une zone de contrôle étant en outre prévue entre les deux zones de test.

15. Trousse de diagnostic selon la revendication 14, **caractérisée en ce que** la zone de contrôle est obtenue à partir de sérumalbumine bovine ou BSA couplée à des particules d'or colloïdales.

16. Trousse de diagnostic pour le dosage simultané de β-lactames, de tétracyclines et de sulfamides, selon l'une quelconque des revendications 9 à 11, **caractérisé en ce que** le mélange réactionnel comprend en outre un anticorps spécifique de la reconnaissance des sulfamides et **en ce que** le système récupérateur comprend en outre une préparation de protéine conjuguée à des sulfamides, immobilisée sur la membrane de nitrocellulose.

17. Trousse de diagnostic pour le dosage simultané de β-lactames, de tétracyclines et de sulfamides, selon la revendication 16, **caractérisée en ce que** la membrane de nitrocellulose, déposée sur un support tigette, est en contact à une première extrémité avec une membrane et à une seconde extrémité avec un papier absorbant et présente trois zones de test distinctes et une zone de contrôle, disposées successivement l'une derrière l'autre dans le sens de migration du liquide.

18. Trousse de diagnostic selon l'une quelconque des revendications précédentes, **caractérisé en ce que** l'échantillon est essentiellement liquide et est obtenu à partir de lait, de miel, de viande, d'oeufs ou de liquides biologiques.

19. Procédé de mise en oeuvre d'une trousse de diagnostic selon l'une quelconque des revendications précédentes, **caractérisé par** les étapes suivantes :
- on met en contact le mélange réactionnel précité avec un échantillon à caractériser pour obtenir une solution qu'on laisse incuber à 50°C pendant 3 minutes ;
- on plonge une tigette portant le système récupérateur précité dans la solution obtenue et on laisse incuber pendant 3 minutes ;
- on interprète quantitativement le résultat sur la tigette au moyen d'un lecteur optique de tigette.
